# EUROPEAN PATENT APPLICATION

(11) **EP 1 157 691 A1**
(43) Date of publication of application: **28.11.2001**
(21) Application number: 00401333.0
(22) Date of filing: 16.05.2000
(51) Int. Cl.: A61K 9/48, A61K 47/36

(54) **Pullulan film compositions with improved surface properties**

(71) Applicant: WARNER-LAMBERT COMPANY, Morris Plains New Jersey 07950 (US)
(72) Inventor: Scott, Robert, 9100 Sint Niklaas (BE); Cade, Dominique, 68000 Colmar (FR); He, Xiongwei, 68280 Andolsheim (FR)
(74) Representative: Tesch, Rudolf, Dr.

(57) **Abstract**

The invention concerns pullulan compositions for the use in pharmaceutical, veterinary, food, cosmetic or other products like films for wrapping food, aspics or jellies, preferably for predosed formulations like soft or hard capsules with improved surface properties obtained by a content of a surfactant of pharmaceutical or food quality.

## Description

The invention concerns pullulan compositions for the use in pharmaceutical, veterinary, food, cosmetic or other products like films for wrapping food, aspics or jellies, preferably for predosed formulations like soft or hard capsules with improved surface properties obtained by a content of a surfactant of pharmaceutical or food quality.

A second embodiment of the invention is the use of the film compositions for the manufacturing of hard capsules by conventional dip moulding process as normally used in the production of conventional hard gelatine capsules. The surfactant in the compositions improves the capsule surface properties in such a way that the capsule works well on the conventional automatic high speed capsule filling equipment.

Conventional hard capsules are made with gelatine by dip moulding process. This process is based on the setting ability of hot gelatine solution by cooling. On a totally automatic industrial hard gelatine capsule machine, the process consists to dip mould pins into hot gelatine solution, to remove the pins from the solution, to turn the pins from downside to upside, to dry the gelatine solution (gel) attached on the pins, to strip the capsule shell and finally to cut and pre-joint the cap and body. The immediate setting of the gelatine solution on the dip pins after dipping is the key step in the process. Otherwise, the gelatine solution would flow down, leading very low top thickness, and no capsule of quality could be produced.

Pullulan is a natural polysaccharide. It has the good film forming property and a particularly low oxygen permeability. Numbers of patents relate to the moulded article in pullulan such as FR 2147112, US 4623394 and US 3784390. Although capsules were mentioned or claimed in these patents, their compositions do not have sufficient setting ability or not at all. Consequently, these compositions do not allow an industrial scale hard capsule production.

To adapt the conventional dip moulding process to pullulan hard capsule production, the inventors proposed in EP 99 40 1849 (application number) compositions containing pullulan and setting system. These compositions make the industrial production of pullulan hard capsules a reality.

However, more investigations revealed that the pullulan hard capsules have poor surface gliding performance, which leads to high opening force of pre-joint capsules and high closing force. Indeed, these are two key parameters for a good filling performance on automatic high speed capsule filling equipment. During the filling process, the filling equipment opens, fills and recloses the capsules in an extremely high cadence. High opening or closing force can lead to defects such as non open, punched capsule ends and etc, and consequently to frequent machine stops.

To resolve this issue, more investigations were made and we have found that addition of small quantity of selected surfactant of pharmaceutical or food quality, we can improve dramatically the pullulan film surface gliding performance, consequently to get the capsule opening and closing forces to the range required by filling equipment.

Therefore, the present invention relates to compositions for hard pullulan capsules with improved surface properties. The compositions are aqueous solutions containing pullulan, setting system and surfactant.

With these aqueous solutions, we can produce hard pullulan capsules with good filling performance by conventional dip mould process just like hard gelatine capsules.

The pullulan in the compositions is the base material for hard capsule making. Its preferred concentration in the aqueous solutions is from 10 to 40%.

The setting system gets the solution to set on the dipped pins, thus assuring a uniform capsule shell thickness. The setting system is preferred to be composed of gelling agent, and optionally salt and sequestering agent.

The preferred gelling agents are kappa-carrageenan and gellan with a concentration in the solutions 0.05-3%.

The salt in the composition is to enhance the setting ability of the gelling agents. It could be salt of cations such as K⁺, Li⁺, Na⁺, NH₄⁺, Ca⁺⁺, Mg⁺⁺ and etc. The preferred salt concentration in the solutions is <2%.

In the case that gellan is used as gelling agent, the compositions can contain optionally sequestering agents to improve the capsule solubility. The preferred sequestering agents are ethylenediaminetetraacetic acid or salts thereof and citric acid and salts thereof. The amount is preferably <1% in the solution compositions.

The surfactant in the compositions is aimed to improve the capsule surface gliding performance, and so the capsule filling performance on filling equipment. The surfactant can be cationic, anionic, non-ionic or amphoteric, and preferably selected from pharmaceutical and food quality such as sodium lauryl sulphate (SLS), dioctyl sodium sulfosuccinate (DSS), benzalkonium chloride, benzethonium chloride, cetrimide (trimethyltetradecylammonium bromide), fatty acid sugar esters, glyceryl monooleate, polyoxyethylene sorbitan fatty acid esters, polyvinyl alcohol, dimethylpolysiloxan, sorbitan esters or lecithin. Its amount based on pullulan is preferably 0.01% to 3%.

The following examples and tests, not limitative, demonstrate the hard capsule making, the surface gliding improvement and the capsule filling performance improvement.

### Example 1: Pullulan film gliding improvement

In 400g of demineralised water at room temperature were dispersed under stirring 0.05 g SLS (500ppm/pullulan), 1 g of kappa-carrageenan (0.2%), 1.25 g of potassium acetate (0.25%) and 100 g of pullulan (20%). Heat the mixture to 70°C under stirring for complete solubilisation and then reduce the stirring for defoaming. The solution then is used to cast on glass plates of 4 mm thickness to form pullulan films of about 100 µm thickness after drying at room conditions.

The pullulan film gliding performance is evaluated by a test on a slanted plan, a method commonly used by gelatine producers. The method determines the smallest angle of inclination of glass plate to provoke the gliding of a film coated glass plate over anther one with films face to face. Consequently, the smaller the gliding angle, the better the film gliding performance.

Repeat the above example with surfactant contents listed in Table 1.

In Table 1, we gathered the gliding performance for different surfactants and quantity.

**Table 1:**

| **Pullulan gliding performance (°)** | | | | |
|---|---|---|---|---|
| **Surfactant** | **No** | **500 ppm** | **1000 ppm** | **5000 ppm** |
| SLS | 29 | 9 | 5 | 6 |
| Hydrolysed deoil lecithin | | 9 | 9 | 7 |
| Polysorbate 20 | | 12 | 12 | |
| Polysorbate 80 | | 10 | 9 | |
| Sorbitan laurate | | 10 | 8 | |
| Sorbitan oleate | | 9 | 7 | |

### Example 2: Pullulan capsule production and performance

In 142 litters of demineralised water at room temperature were dispersed under stirring 20 g hydrolysed deoil lecithin (500ppm/pullulan), 363 g kappa-carrageenan (0.2%) and 40 kg pullulan (22%). Heat the mixture up to 70°C under stirring for total solubilisation. 455 g potassium acetate previously dissolved in some water was then added into the solution. A slurry made with 800 g TiO₂, 3 litres hot water and 3 litres so prepared pullulan solution by high shearing was added into the solution in order to produce white opaque capsules. After defoaming, the solution is finally stabled at 60°C.

A second identical preparation was made. The two preparations were used to feed a conventional hard gelatine capsule production machine, white opaque hard pullulan capsules were then produced in the similar way to hard gelatine capsules.

As reference, transparent pullulan hard capsules without surfactant in the formulation were produced in the same way as above.

The improvement of hard pullulan capsules by the addition of surfactant is illustrated by the data gathered in Table 2, and was confirmed by a filling trial on a filling equipment KGF400.

**Table 2**

| Capsule | Opening force of pre-lock capsule | Closing force |
|---|---|---|
| Capsule of example 2 | 12 g | 6.0 N |
| Reference capsule | 36 g | 7.6 N |

## Claims

1. Film forming compositions consisting of pullulan and a setting system **characterised in that** the composition contains one or more surfactant.

2. Film forming compositions according to claim 1, wherein the surfactant is selected from sodium lauryl sulphate (SLS), dioctyl sodium sulfosuccinate (DSS), benzalkonium chloride, benzethonium chloride, cetrimide (trimethyltetradecylammonium bromide), fatty acid sugar esters, glyceryl monooleate, polyoxyethylene sorbitan fatty acid esters, polyvinyl alcohol, dimethylpolysiloxan, sorbitan esters or lecithin.

3. Film forming compositions according to claim 1 or 2, wherein the content of surfactant is 0.01 to 3 % by weight related to the amount of pullulan.

4. Containers for unit dosage forms for agrochemicals, seeds, herbs, foodstuffs, dyestuffs, pharmaceuticals, or flavouring agents produced from the compositions according to claims 1 to 3.

5. Container according to claim 4 which is a pharmaceutical capsule.

6. Caplets encapsulated in film forming compositions according to claims 1 to 3.

7. Capsules according to claim 4 to 6 **characterised in that** the capsule halves are sealed with one or more layers of the composition according to claims 1 to 3.

8. Capsules according to claim 4 to 6 **characterised in that** a liquid fusion process seals the capsule halves.

9. Capsules according to claim 4 to 6 **characterised by** a release of filled product at low temperature, such as at room temperature.

10. Aqueous solutions of compositions according to claims 1 to 3 for the manufacturing of capsules.

11. Use of aqueous solutions according to claim 10 for the manufacturing of hard capsules in a dip moulding process.

12. Manufacturing of hard capsules from aqueous pullulan solutions according to claim 10 in a dip moulding process with conventional hard gelatine capsules process parameters and equipment.
